# EUROPEAN PATENT SPECIFICATION

(11) **EP 4 140 508 B1**
(45) Date of publication and mention of the grant of the patent: **03.04.2024**
(21) Application number: 21197057.9
(22) Date of filing: 16.09.2021
(51) Int. Cl.: A61L 9/013, A61L 9/12, A61L 9/20, A61L 9/015, F24F 8/22, F24F 8/26

(54) **APPARATUS FOR MEASURING LIQUID LEVEL AND AIR STERILIZER HAVING THE SAME**
VORRICHTUNG ZUR MESSUNG DES FLÜSSIGKEITSNIVEAUS UND LUFTSTERILISATOR DAMIT
APPAREIL DE MESURE DE NIVEAU DE LIQUIDE ET STÉRILISATEUR D'AIR LE COMPRENANT

(30) Priority: 24.08.2021 KR 20210111384; 24.08.2021 KR 20210111393
(43) Date of publication of application: 01.03.2023
(73) Proprietor: Wellis Co. Ltd., Seoul (KR)
(72) Inventor: Yoo, EuiSeok, Goyang-si (KR); Yim, NohBin, Seoul (KR)
(74) Representative: Botti & Ferrari S.p.A.

(56) References cited:
- WO-A1-2017/090821
- KR-A- 20150 010 009
- US-A1- 2014 079 597
- US-A1- 2021 236 683
- Unknown: "Wellis 2020 Wadu 02 Wadu 03", , 20 June 2020 (2020-06-20), pages 1-5, XP055896893, Retrieved from the Internet: URL:https://www.youtube.com/watch?v=5YmdRz oln_w [retrieved on 2022-03-02]

## Description

### BACKGROUND OF THE INVENTION

### FIELD OF THE INVENTION

The present invention relates to an apparatus for measuring liquid level and an air sterilizer having the apparatus for measuring liquid level.

### DISCUSSION OF THE BACKGROUND

As industrialization progresses and as urbanization and population densification accelerate, the problem of air pollution that humans breathe every day is getting more serious. Fossil fuels that humans use every day inevitably emit pollutants harmful to humans into the air, and the concentration of pathogenic fine substances such as viruses and bacteria is also increasing in the air, so that humans' needs to breathe clean air is getting stronger. A device used to satisfy these needs is an air purifier or air sterilizer.

Most pollutants in the air are inhaled by humans through respiration to act as a cause of various diseases. Hydroxyl radical (OH) is a major air cleaning agent that removes various pollutants such as carbon monoxide, sulfur dioxide, and nitrogen dioxide.

In order to generate hydroxyl radical (OH) as described above, ozone and evaporated hydrogen peroxide are reacted or ozone and evaporated limonene solution are reacted. When the complete reaction does not occur, consumption of the reactant will increase. In addition, remaining ozone emitted into the atmosphere may have adverse effects on the human body, so improvement is needed.

Examples of ozone sterilizing devices are disclosed in WO2017090821, XP055896893, US2014079597, US2021236683 and KR20150010009.

### SUMMARY OF THE INVENTION

Therefore, the problem to be solved by the present invention is to provide an air sterilizer capable of increasing the generation efficiency of hydroxyl radicals.

The air sterilizer includes the features of claim 1.

As an embodiment, the first air path and the second air path may not be parallel to each other.

As an embodiment, the first air path and the second air path may be disposed perpendicular to each other.

As an embodiment, the first air path may have a portion with relatively smaller diameter comparing to other portions, with which an inlet of the container is in contact, so that liquid in the container can be sucked up.

As an embodiment, the air sterilizer may further include a UV LED disposed in the third air path.

As an embodiment, at least a portion of inner wall of the third air path may be formed with a TiO₂ or a Delafosite (iron-copper alloy) mesh coating.

As an embodiment, the air sterilizer may further include a blowing fan driving motor rotating the blowing fan, and the mixing fan may be rotated by air introduced through the second air path.

In this case, a diameter of the second air path may be gradually decreased to be connected to the mixing space, to increase a speed of air flowing into the mixing space from the second air path in order to increase rotation speed of the mixing fan.

As an embodiment, the air sterilizer may further include a blowing fan driving motor rotating the blowing fan, and a mixing fan driving motor rotating the mixing fan.

In this case, the air sterilizer may further include a controller individually controlling rotational speed of the blowing fan driving motor and rotational speed of the mixing fan driving motor to control mixing ratio of air containing evaporated liquid in the container, which passes through the first air path to arrive at the mixing space, and air containing ozone, which passes through the second air path to arrive at the mixing space.

In this case, the container may contain limonene solution (olefin), and the controller may control speed of the blowing fan driving motor and the mixing fan driving motor individually such that a ratio of ozone : limonene is in a range of 1 : 0.3 ~ 1 : 3.

Alternatively, the container may contain hydrogen peroxide, and the controller may control speed of the blowing fan driving motor and the mixing fan driving motor individually such that a ratio of ozone : hydrogen peroxide is in a range of 1 : 0.3 ~ 1 : 2.

As an embodiment, the mixing fan may be disconnectably connected to the mixing fan driving motor.

As an embodiment, the container may be detachable from the housing.

As an embodiment, the air sterilizer may further include an apparatus for measuring liquid level for measuring remaining amount of liquid in the container.

The apparatus for measuring liquid level may include a light emitting device, and a light receiving device disposed at least one of a first position, at which a light generated by the light emitting device passes through a container with liquid to arrive, and a second position, at which a light generated by the light emitting device passes through an empty container to arrive.

For example, the light emitting device and the light receiving device may be arranged at a reference height in which a required minimum liquid is contained.

As an embodiment, a number of the light emitting device and the light receiving device may be plural, and the light emitting devices and the light receiving devices may be arranged in the Z-axis direction (gravity direction).

As an embodiment, the light emitting device may generate a light of which cross-section has a vertical linear shape.

As an embodiment, the apparatus for measuring liquid level may further include a lens changing a light of which a beam has a point-shape, which is generated by the light emitting device, to be a vertical linear shape.

As an embodiment, a position of at least one of the light emitting device and the light receiving device may be changeable.

As described above, according to the air sterilizer of the present invention, the first air path and the second air path are not parallel to each other, but are vertically arranged, so that ramonene or hydrogen peroxide and ozone can be mixed completely, thereby increasing the generation of hydroxyl radicals.

In addition, through UV LED, it is possible to proceed with additional sterilization in the discharged air, and through UV LED and TiO₂ or Delafosite coating, it is possible to generate hydroxyl radical (OH) and amplify its energy.

In addition, when only the blowing fan is driven by the blowing fan driving motor and the mixing fan is rotated by the air that flows through the second air path AP2 forcibly by the blowing fan, energy can be relatively reduced. On the other hand, when the blowing fan is driven by the blowing fan driving motor and the mixing fan is driven by the mixing fan driving motor, respectively, the consumption of reactants is minimized by allowing the reaction to react through the optimum reaction ratio between ozone and hydrogen peroxide or evaporated limonene solution. In addition, it is possible to efficiently generate hydroxyl radicals while suppressing the emission of ozone.

In addition, when the mixing fan of the air sterilizer is driven by the mixing fan driving motor, and the mixing fan and the mixing fan driving motor are disconnectably connected, the above two advantages can be selected.

In addition, when the container is formed to be detachable from the housing, it is easier to inject hydrogen peroxide or limonene solution into the container.

In addition, when the air sterilizer further includes an apparatus for measuring liquid level for measuring the remaining amount of liquid in the container, it is possible to check the consumption state of hydrogen peroxide or limonene solution in the container.

### BRIEF DESCRIPTION OF THE DRAWINGS

The accompanying drawings, which are included to provide a further understanding of the invention and are incorporated in and constitute a part of this specification, illustrate embodiments of the invention, and together with the description serve to explain the principles of the invention
FIG. 1 is a perspective view of an air sterilizer according to an exemplary embodiment of the present invention.
FIG. 2 is a front cross-sectional view of the air sterilizer shown in FIG. 1.
FIG. 3 is a front cross-sectional view of an air sterilizer according to another exemplary embodiment of the present invention.
FIG. 4 is a view showing a simulation result showing the air flow in the mixing space of the air sterilizer according to the present invention.
FIG. 5 is a perspective view of an apparatus for measuring liquid level according to an exemplary embodiment of the present invention, and the apparatus may be applied to the air sterilizer of FIGS. 2 and 3.
FIG. 6 is a plan view illustrating a state in which liquid is empty in FIG. 5.
FIG. 7 is a plan view illustrating a state in which liquid is filled in FIG. 5.
FIG. 8 is a plan view illustrating an apparatus for measuring liquid level according to another exemplary embodiment of the present invention, and the apparatus may be applied to the air sterilizer of FIGS. 2 and 3.
FIG. 9 is a front view of the apparatus for measuring liquid level shown in FIGS. 5 to 7 or 8.
FIG. 10 is a front view showing an apparatus for measuring liquid level according to still another exemplary embodiment of the present invention, and the apparatus may be applied to the air sterilizer of FIGS. 2 and 3.
FIG. 11 is a front view showing an apparatus for measuring liquid level according to still another exemplary embodiment of the present invention, and the apparatus may be applied to the air sterilizer of FIGS. 2 and 3.
FIG. 12 is a plan view showing an apparatus for measuring liquid level according to still another exemplary embodiment of the present invention, and the apparatus may be applied to the air sterilizer of FIGS. 2 and 3.

### DETAILED DESCRIPTION OF THE ILLUSTRATED EMBODIMENTS

The present invention is described more fully hereinafter with reference to the accompanying drawings, in which example embodiments of the present invention are shown. The present invention may, however, be embodied in many different forms and should not be construed as limited to the example embodiments set forth herein. Rather, these example embodiments are provided so that this disclosure will be thorough and complete, and will fully convey the scope of the present invention to those skilled in the art. In the drawings, the sizes and relative sizes of layers and regions may be exaggerated for clarity.

Hereinafter, exemplary embodiments of the present invention will be described in detail with reference to the accompanying drawings.

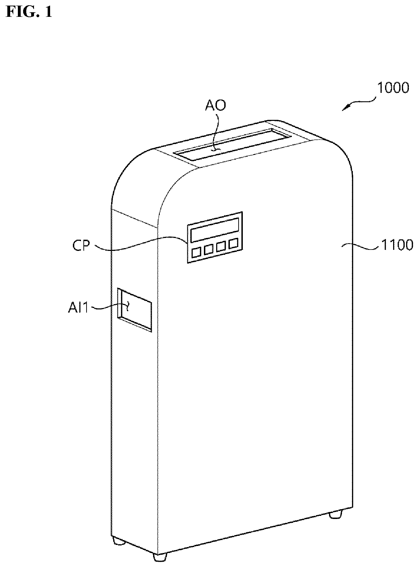

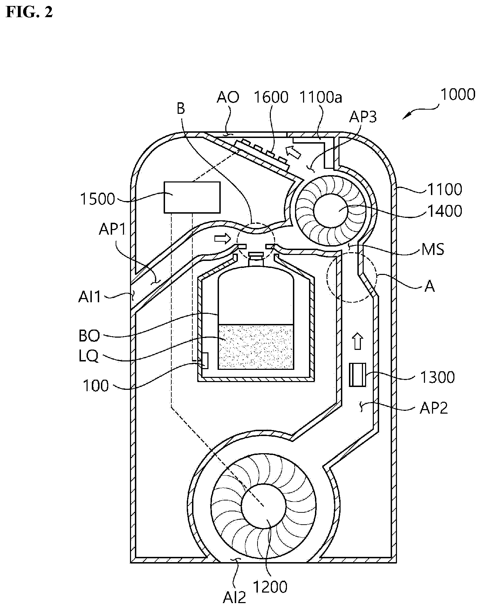

FIG. 1 is a perspective view of an air sterilizer according to an exemplary embodiment of the present invention, and FIG. 2 is a front cross-sectional view of the air sterilizer shown in FIG. 1.

Referring to FIGS. 1 and 2, an air sterilizer 1000 according to an exemplary embodiment of the present invention includes a housing 1100, a container BO, a blowing fan 1200, an ozone generator 1300, and a mixing fan 1400. As an embodiment, the air sterilizer 1000 may further include a controller 1500 for controlling the air sterilizer 1000, and an apparatus for measuring liquid level 100 for measuring the remaining amount of liquid in the container BO.

The housing 1100 is, for example, a stand type, and may have legs or wheels at the bottom. Alternatively, the housing 1100 may be a wall-mounted type to be hung on the wall.

A control panel CP may be provided on the surface of the housing 1100. The control panel CP is linked with the controller 1500, and a user can control the operation of the air sterilizer 1000 through the control panel CP. Additionally, the control panel CP may display remaining amount of liquid in the container BO sensed through the apparatus for measuring liquid level 100.

A first air inlet AI1 and a second air inlet AI2 through which external air is sucked are provided on the surface of the housing 1100, and an air outlet AO for discharging air containing hydroxyl radicals is provided on the surface of the housing 1100.

Inside the housing 1100, a first air path AP1, a second air path AP2 and a third air path AP3 are formed. Through the first air path AP1, air mixed with evaporated limonene or hydrogen peroxide flows, and through the second air path AP2, air containing ozone flows, and through the third air path, hydroxyl radicals flow. In addition, the first air path AP1 and the second air path AP2 are connected to the mixing space MS, so that limonene or hydrogen peroxide evaporated is mixed with ozone in the mixing space MS to generate hydroxyl radicals.

That is, the first air path AP1 connects between the first air inlet AI1 and the mixing space MS. The second air path AP2 connects between the second air inlet AP2 and the mixing space MS. The third air path AP3 connects between the mixing space MS and the air outlet AO.

The first air path AP1 and the second air path AP2 may be disposed not in parallel with each other. For example, the first air path AP1 and the second air path AP2 may be disposed perpendicular to each other.

As in the prior art, when the first air path AP1 through which the evaporated limonene or hydrogen peroxide flows and the second air path AP2 through which air with ozone are formed in a parallel direction, so that the evaporated limonene or hydrogen peroxide is mixed with ozone, the flow of the evaporated limonene or hydrogen peroxide and the flow of the air with ozone becomes a laminar flow. Therefore, ozone is not fully reacted with limonene or hydrogen peroxide, and thus unreacted ozone may be discharged, and total amount of generated hydroxyl radical becomes relatively smaller, comparing consumed limonene or hydrogen peroxide.

However, when the first air path AP1 and the second air path AP2 are not in parallel with each other, or when the first air path AP1 and the second air path AP2 are disposed perpendicular to each other, turbulence flow may be induced, thereby improving mixing efficiency.

The container BO contains the liquid, and is disposed inside the housing 1100 such that the inlet of the container BO is in contact with the first air path AP1. In one embodiment, the container BO may be detachable from the housing 1100.

The container BO is formed of a transparent material, therefore, the remaining amount of liquid LQ in the container BO can be sensed through the apparatus for measuring liquid level 100 disposed outside of the container BO.

The blowing fan 1200 is disposed in the second air path AP2, and sucks air from the second air inlet AI2.

The ozone generator 1300 is disposed in the second air path AP2, and generates ozone using air introduced through the second air inlet AI2.

The mixing fan 1400 is placed in the mixing space MS, and mixes air containing evaporated liquid in the container BO and flowing through the first air path AP1 to arrive at the mixing space MS and air containing ozone and flowing through the second air path AP2 to arrive at the mixing space MS.

As an embodiment, the first air path AP1 has a portion (region B in FIG. 2) with relatively smaller diameter comparing to other portions, with which the inlet of the container BO is in contact, so that liquid LQ in the container BO can be sucked up.

As an embodiment, the air sterilizer 1000 may further include a UV LED 1600 disposed in the third air path AP3.

As an embodiment, the air sterilizer 1000 may further include the TiO₂, Delafosite (iron-copper alloy) mesh coating 1100a or other metal oxides with the ability to absorb in the visible spectrum as metal nitrides (titanium, chromium ...), strontium titanate, modified with transition metal dimers and trimers (for example using glycine, defienylamine, or p-amino benzoic acid ), which is formed on at least a portion of the inner wall of the third air path AP3.

Therefore, through UV LED, it is possible to further sterilize the discharged air, and through UV LED and TiO₂ or Delafosite coating 1 100a, it is possible to generate more hydroxyl radical (OH) and amplify its energy.

As an embodiment, the air sterilizer 1000 may further include a blowing fan driving motor (not shown) that rotates the blowing fan 1200, and the mixing fan 1400 can be rotated by air flowing through the second air path AP2 to arrive at the mixing space MS.

In this case, the diameter of the second air path AP2 may be gradually decreased to be connected to the mixing space MS (region A in FIG. 2), to increase the speed of air flowing into the mixing space MS from the second air path AP2.

The controller 1500 may be electrically connected to the blowing fan driving motor (not shown) and the apparatus for measuring liquid level 100 and the UV LED 1600 to control them.

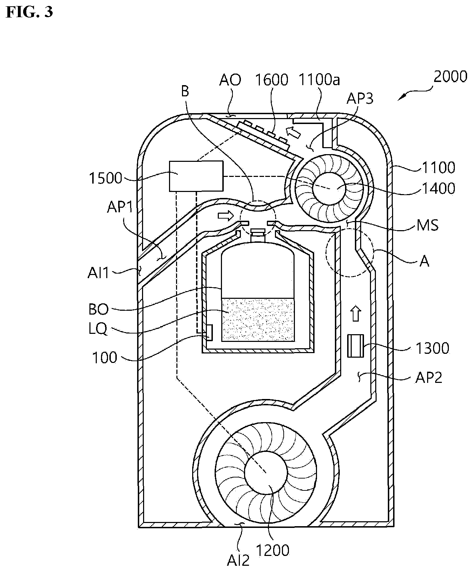

FIG. 3 is a front cross-sectional view of an air sterilizer according to another exemplary embodiment of the present invention. The air sterilizer 2000 in FIG. 3 is substantially same as the air sterilizer 1000 in FIG. 2, except that the air sterilizer 2000 further includes a mixing fan driving motor for driving the mixing fan 1400 and the controller 1500 controls the mixing fan driving motor. Therefore, the same or similar components are denoted by the same reference numerals, and any further descriptions will be omitted.

Referring to FIG. 3, an air sterilizer 2000 according to an exemplary embodiment of the present invention includes a housing 1100, a container BO, a blowing fan 1200, an ozone generator 1300 and a mixing fan 1400. In addition, the air sterilizer 2000 further includes a blowing fan driving motor (not shown) that rotates the blowing fan 1200 and a mixing fan driving motor (not shown) that rotates the mixing fan 1400.

The air sterilizer 2000 may further include a controller 1500 controlling a speed of the blowing fan driving motor (not shown) and a speed of the mixing fan driving motor (not shown), individually to control mixing ratio of air containing evaporated liquid LQ in the container BO, which passes through the first air path AP1 to arrive at the mixing space MS, and air containing ozone, which passes through the second air path AP2 to arrive at the mixing space MS.

For example, the container BO may contain limonene solution (olefin), and the controller 1500 may control the speed of the blowing fan driving motor (not shown) and the mixing fan driving motor (not shown) individually such that a ratio of ozone : limonene is in a range of 1 : 0.3 ~ 1 : 3.

Alternatively, the container BO may contain hydrogen peroxide, and the controller 1500 may control the speed of the blowing fan driving motor (not shown) and the mixing fan driving motor (not shown) individually such that a ratio of ozone : hydrogen peroxide is in a range of 1 : 0.3 ~ 1 : 2.

As such, when the mixing fan 1400 of the air sterilizer 2000 is driven by the mixing fan driving motor (not shown), the air sterilizer 2000 can be formed such that the mixing fan 1400 and the mixing fan driving motor (not shown) may be disconnectably connected. Therefore, when the mixing fan driving motor (not shown) is disconnected with the mixing fan 1400, energy consumption can be reduced by driving only the blowing fan driving motor. On the other hand, when the mixing fan driving motor (not shown) is connected with the mixing fan 1400, limonene or hydrogen peroxide can be optimally mixed with ozone to reduce consumption of the limonene or hydrogen peroxide and to minimize amount of discharged ozone.

During manufacturing of the air sterilizer 2000, the rotational speed of the blowing fan 1200 and the mixing fan 1400 may be set. Alternatively, during operation of the air sterilizer 2000, the rotation speed of the blowing fan 1200 and the mixing fan 1400 may be controlled, respectively, by sensing the amount of limonene or hydrogen peroxide and the amount of ozone, respectively.

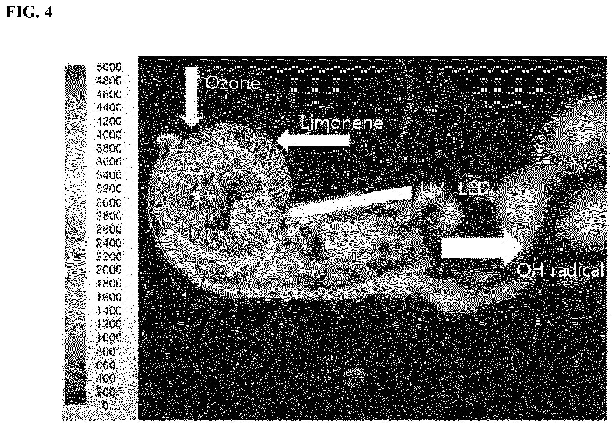

FIG. 4 is a view showing a simulation result showing the air flow in the mixing space of the air sterilizer according to the present invention.

Referring to FIG. 4, since the first air path and the second air path are vertically arranged, generation of turbulence can be confirmed, and through the turbulence, limonene and ozone can be mixed more efficiently.

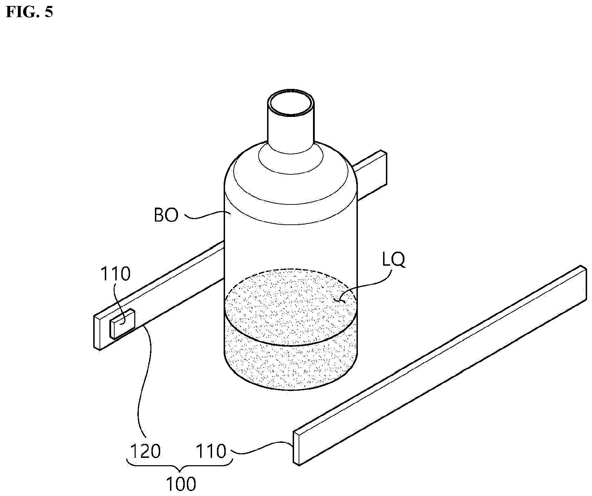

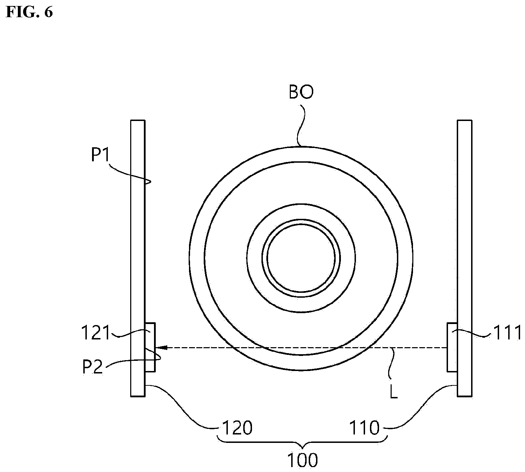

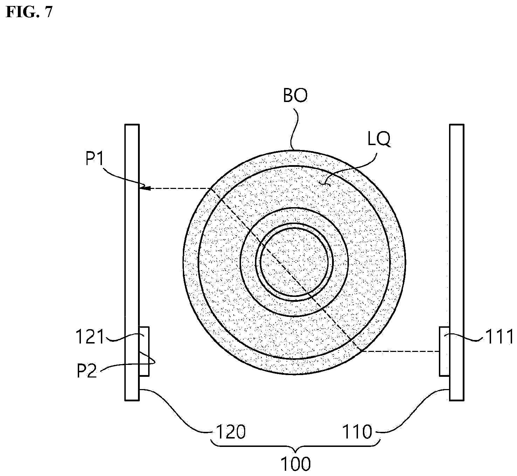

FIG. 5 is a perspective view of an apparatus for measuring liquid level according to an exemplary embodiment of the present invention, and the apparatus may be applied to the air sterilizer of FIGS. 2 and 3. FIG. 6 is a plan view illustrating a state in which liquid is empty in FIG. 5, and FIG. 7 is a plan view illustrating a state in which liquid is filled in FIG. 5.

Referring to FIGS. 5 to 7, an apparatus for measuring liquid level 100 according to an exemplary embodiment of the present invention includes a light emitting device 111 and a light receiving device 121.

The light emitting device 111 may be mounted on the light emitting device driving substrate 110, and the light receiving device 121 may be mounted on the light receiving device driving substrate 120. For convenience, the light emitting device driving substrate 110 and the light receiving device driving substrate 120 are illustrated as separate substrates, but may be configured as one substrate through a flexible circuit board or the like.

The light emitting device 111 may be, for example, an LED or a laser diode, and the light receiving device 121 may be an optical sensor such as a photodiode.

The light receiving device 121 is disposed at least one of a first position P1, at which a light generated by the light emitting device 111 passes through a container with liquid to arrive, and a second position P2, at which a light generated by the light emitting device 111 passes through an empty container to arrive. For example, in this embodiment, the light receiving device 121 is disposed at the second position P2.

The light emitting device 111 is disposed such that incident angle of the light emitted from the light emitting device 111 is not perpendicular to the surface of the container BO. Accordingly, a light L emitted from the light emitting device 111 and incident on the container BO having liquid LQ is refracted and proceeds to the first position P1 as shown in FIG. 3, but a light L emitted from the light emitting device 111 and incident on the container BO without liquid LQ proceeds straight to the second position P2 as shown in FIG. 2 to be sensed by the light receiving device 121. On the other hand, although some refraction is made by the container BO including plastic or glass, the refraction due to the container BO is ignored in FIG. 2, since the thickness of the container is thin.

Therefore, in this embodiment, when light sensing is not performed by the light receiving device 121, it can be considered that the liquid LQ is filled in the container BO, and when light sensing is performed by the light receiving device 121, it can be considered that the liquid LQ is not filled in the container BO.

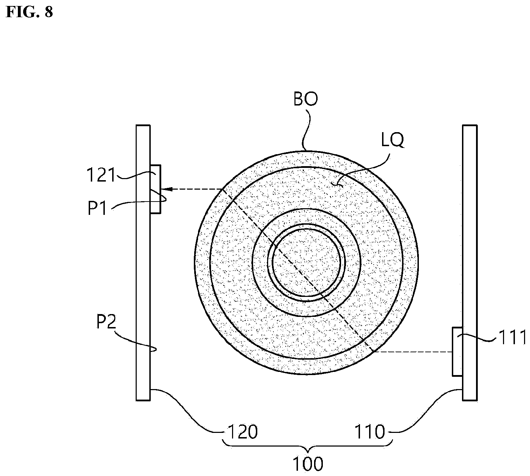

FIG. 8 is a plan view illustrating an apparatus for measuring liquid level according to another exemplary embodiment of the present invention, and the apparatus may be applied to the air sterilizer of FIGS. 2 and 3. The apparatus for measuring liquid level disclosed in FIG. 8 is substantially same as the apparatus for measuring liquid level disclosed in FIGS. 5 to 7 except for the position of the light receiving device. Accordingly, the same or similar components are denoted by the same reference numerals, and any further explanation will be omitted.

Referring to FIG. 8, according to an apparatus for measuring liquid level according to another exemplary embodiment of the present invention, the light receiving device is disposed at the first position P1.

Therefore, in this embodiment, when light sensing is performed by the light receiving device 121, it can be considered that the solution LQ is filled in the container BO, and when light sensing is not performed by the light receiving device 121, it can be considered that the solution LQ is not filled in the container BO.

On the other hand, in the previous embodiments, the light receiving device 121 is provided only in the first position P1 or the second position P2, but two light receiving devices 121 may be provided in both the first position P1 and the second position P2, respectively. When light sensing is performed by the light receiving device 121 in the first position P1, it can be considered that the solution LQ is filled in the container BO, and when light sensing is performed by the light receiving device 121 in the second position P2, it can be considered that the solution LQ is not filled in the container BO.

When the light receiving device 121 is provided at the first position P1 or the second position P2 only, in case that a failure occurs in the light receiving device 121 or its driving device, it may be difficult to detect the failure. However, when two light receiving devices 121 are provided at the position P1 and the second position P2, respectively, the failure can be detected.

As described above, according to the present invention, it is possible to detect whether the container BO contains liquid LQ or not, by using the difference in refractive index between the case in which the solution LQ is filled in the container BO and the case in which the solution LQ is not filled in the container BO.

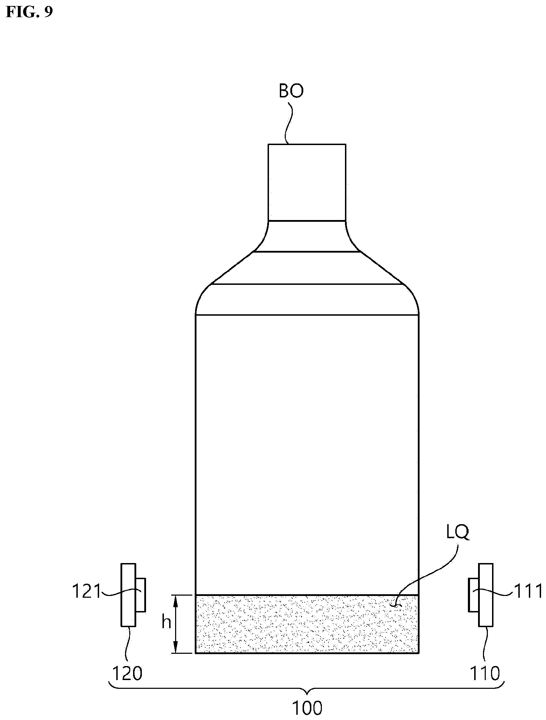

FIG. 9 is a front view of the apparatus for measuring liquid level shown in FIGS. 5 to 7 or 8.

Referring to FIGS. 5 to 9, the light emitting device 111 and the light receiving device 121 may be disposed at a reference height h in which a required minimum liquid is contained. Therefore, when the solution in the container BO is gradually reduced to reach the reference height h, it can be detected by the light receiving device 121.

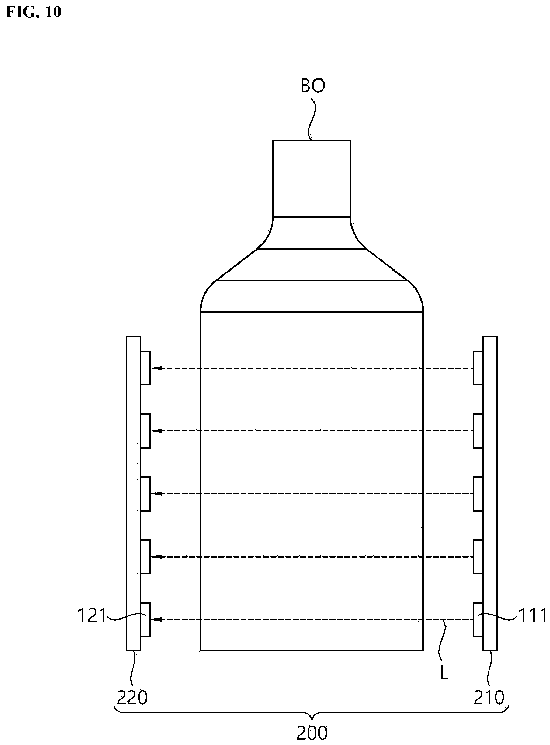

FIG. 10 is a front view showing an apparatus for measuring liquid level according to still another exemplary embodiment of the present invention, and the apparatus may be applied to the air sterilizer of FIGS. 2 and 3. The apparatus for measuring liquid level 200 disclosed in FIG. 10 is substantially same as the apparatus for measuring liquid level 100 disclosed in FIGS. 5 to 7 or in FIG. 8 except for the number of the light emitting device 111 and the light receiving device 121. Therefore, same or similar components are denoted by the same reference numerals, and any further explanation will are omitted.

Referring to FIG. 10, an apparatus for measuring liquid level 200 according to another exemplary embodiment of the present invention includes a plurality of light emitting devices 111 and a plurality of light receiving devices 121 arranged in the Z-axis direction (gravity direction). The plurality of light emitting devices 111 and the plurality of light receiving devices 121 are arranged to be one-to-one matching in the Z-axis direction. However, as described above, one of the plurality of light emitting devices 111 may correspond to two light receiving devices 121 of the plurality of light receiving devices 121, disposed at the position P1 and the second position P2, respectively.

When only one light emitting device 111 and one light receiving device 121 or two light receiving devices 121 in the first position P1 and the second position P2 are provided, it is possible to detect that liquid in the container BO reaches the reference position h in FIG. 9. However, as in the embodiment of FIG. 10, when a plurality of light emitting devices 111 and light receiving devices 121 arranged in the Z-axis direction are provided, a level of the liquid LQ in the container BO can be detected.

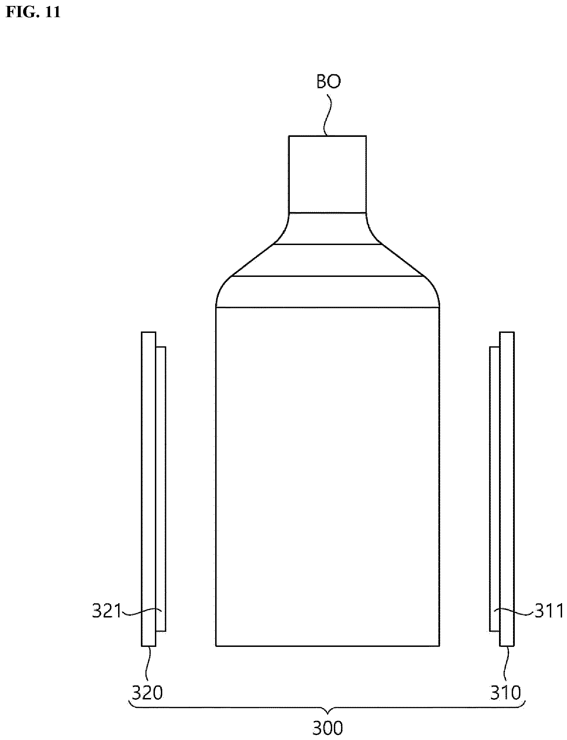

FIG. 11 is a front view showing an apparatus for measuring liquid level according to still another exemplary embodiment of the present invention, and the apparatus may be applied to the air sterilizer of FIGS. 2 and 3. The apparatus for measuring liquid level 300 disclosed in FIG. 11 is substantially the same as the apparatus for measuring liquid level 200 disclosed in FIG. 10 except for the number and shape of a light emitting device and a light receiving device. Therefore, same or similar components are denoted by the same reference numerals, and any further explanation will be omitted.

Referring to FIG. 11, an apparatus for measuring liquid level 300 according to another exemplary embodiment of the present invention includes a light emitting device 311 and a light receiving device 321.

The light emitting device 311 may be mounted on the light emitting device driving substrate 310, and the light receiving device 321 may be mounted on the light receiving device driving substrate 320. For convenience, the light emitting device driving substrate 310 and the light receiving device driving substrate 320 are illustrated as separate substrates, but may be configured as one substrate through a flexible circuit board or the like.

The light emitting device 311 may emit light of which cross-section has a vertical linear shape. That is, the light emitting device 311 may generate line-shaped light.

As an embodiment for this, the light emitting device 311 may emit a light having a point-shaped cross-section, and a lens may change the light to be a light of which cross-section has a vertical linear shape. That is, the light emitting device 311 may generate a line beam by itself, or may convert a point light source into a line beam using a lens.

Also, the light receiving device 321 may be an element for receiving a line-shaped beam. The light receiving device 321 may convert the amount of received into an electric current, so that it is possible to read out the level of liquid continuously.

That is, the apparatus for measuring liquid level 200 shown in FIG. 10 can measure the level of the liquid LQ discontinuously, but the apparatus for measuring liquid level 300 shown in FIG. 11 can measure the level of the liquid LQ continuously.

Meanwhile, in this embodiment, the light emitting device 311 and the light receiving device 321 are both configured to generate a line beam and receive the line beam, respectively, but one of the plurality of light emitting devices 311 and the plurality of light receiving devices 321 in FIG. 10 may replace one of the light emitting device 111 or the light receiving device 121 in FIG. 11, respectively.

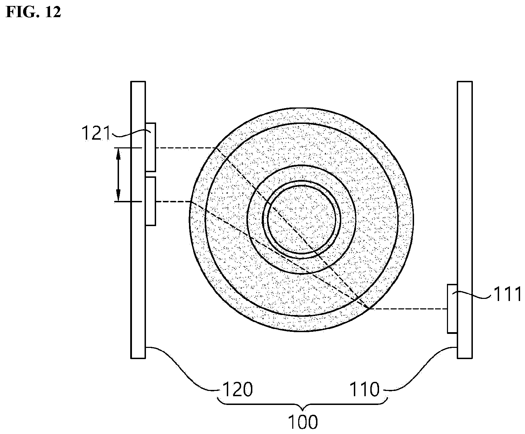

FIG. 12 is a plan view showing an apparatus for measuring liquid level according to still another exemplary embodiment of the present invention, and the apparatus may be applied to the air sterilizer of FIGS. 2 and 3. The apparatus for measuring liquid level disclosed in FIG. 12 is substantially same as the apparatus for measuring liquid level disclosed in FIG. 8 except that the position of the light receiving device can be changed. Therefore, same or similar components are denoted by the same reference numerals, and any further explanations will be omitted.

Referring to FIG. 12, the apparatus for measuring liquid level according to another exemplary embodiment of the present invention is configured to have a function that the position of the light receiving device 121 is changed.

Accordingly, when the solution LQ in the container BO is changed, the refractive index is also changed, and thus, the present embodiment can respond to this.

Meanwhile, in the present embodiment, the position of the light receiving device 121 can be changed, but it can also possible to change the position of the light emitting device 111. Further, the position of both of the light emitting device 111 and the light receiving device 121 can be changed.

As described above, the apparatus for measuring liquid level according to the present invention can measure the remaining amount of liquid contained in a transparent container.

It will be apparent to those skilled in the art that various modifications and variation may be made in the present invention without departing from the scope of the claims.

## Claims

1. An air sterilizer (1000, 2000) comprising:
a housing (1100) in which a first air path (AP1) connecting a first air inlet (AI1) and a mixing space (MS), a second air path (AP2) connecting a second air inlet (AI2) and the mixing space (MS), and a third air path (AP3) connecting the mixing space (MS) and an air outlet (AO) are formed;
a container (BO) disposed inside the housing (1100) to hold a liquid (LQ), an inlet of the container (BO) being in contact with the first air path (AP1);
a blowing fan (1200) disposed in the second air path (AP2), the blowing fan (1200) enforcing air to flow from the second air inlet (AI2) to the mixing space (MS);
an ozone generator (1300) disposed in the second air path (AP2) to generate ozone using air introduced through the second air inlet (AI2); and
**characterised by**
a mixing fan (1400) disposed in the mixing space (MS), the mixing fan (1400) mixing air containing evaporated liquid in the container (BO) and flowing through the first air path (AP1) to arrive at the mixing space (MS) and air containing ozone and flowing through the second air path (AP2) to arrive at the mixing space (MS).

2. The air sterilizer of claim 1, wherein the first air path (AP1) and the second air path (AP2) are not parallel to each other.

3. The air sterilizer of claim 1, wherein the first air path (AP1) and the second air path (AP2) are disposed perpendicular to each other.

4. The air sterilizer of claim 1, wherein the first air path (AP1) has a portion with relatively smaller diameter comparing to other portions, with which an inlet of the container (BO) is in contact, so that liquid in the container (BO) can be sucked up.

5. The air sterilizer of claim 1, further comprising:
a UV LED (1600) disposed in the third air path (AP3), and, wherein at least a portion of inner wall of the third air path (AP3) is formed with a TiO₂ or Delafosite coating (1100a).

6. The air sterilizer of claim 1, further comprising:
a blowing fan driving motor rotating the blowing fan; and
wherein the mixing fan (1400) is rotated by air introduced through the second air path (AP2).

7. The air sterilizer of claim 6, wherein a diameter of the second air path (AP2) is gradually decreased to be connected to the mixing space (MS), to increase a speed of air flowing into the mixing space (MS) from the second air path (AP2) in order to increase rotation speed of the mixing fan (1400).

8. The air sterilizer of claim 1, further comprising:
a blowing fan driving motor rotating the blowing fan (1200); and
a mixing fan driving motor rotating the mixing fan (1400).

9. The air sterilizer of claim 8, further comprising:
a controller (1500) individually controlling rotational speed of the blowing fan driving motor and rotational speed of the mixing fan driving motor to control mixing ratio of air containing evaporated liquid in the container (BO), which passes through the first air path (AP1) to arrive at the mixing space (MS), and air containing ozone, which passes through the second air path (AP2) to arrive at the mixing space (MS).

10. The air sterilizer of claim 9, wherein the container (BO) contains limonene solution (olefin), and
the controller (1500) controls speed of the blowing fan driving motor and the mixing fan driving motor individually such that a ratio of ozone : limonene is in a range of 1 : 0.3 ~ 1 : 3.

11. The air sterilizer of claim 9, wherein the container (BO) contains hydrogen peroxide, and
the controller (1500) controls speed of the blowing fan driving motor and the mixing fan driving motor individually such that a ratio of ozone : hydrogen peroxide is in a range of 1 : 0.3 ~ 1 : 2.

12. The air sterilizer of claim 8, wherein the mixing fan (1400) is disconnectably connected to the mixing fan driving motor.

13. The air sterilizer of claim 1, further comprising:
an apparatus for measuring liquid level (100) for measuring remaining amount of liquid in the container, wherein the apparatus for measuring liquid level (100) comprises:
a light emitting device (111); and
a light receiving device (121) disposed at least one of a first position (P1), at which a light generated by the light emitting device (111) passes through a container (BO) with liquid (LQ) to arrive, and a second position (P2), at which a light generated by the light emitting device (111) passes through an empty container (BO) to arrive.

14. The air sterilizer of claim 13, wherein a number of the light emitting device (111) and the light receiving device (121) are plural, and
the light emitting devices (111) and the light receiving devices (121) are arranged in the Z-axis direction (gravity direction).

15. The air sterilizer of claim 13, wherein the light emitting device (311) generates a light of which cross-section has a vertical linear shape.

## Patentansprüche

1. Luftsterilisator (1000, 2000) umfassend:
ein Gehäuse (1100), in dem ein erster Luftpfad (AP1), der einen ersten Lufteinlass (AI1) und einen Mischraum (MS) verbindet, ein zweiter Luftpfad (AP2), der einen zweiten Lufteinlass (AI2) und den Mischraum (MS) verbindet, und ein dritter Luftpfad (AP3), der den Mischraum (MS) und einen Luftauslass (AO) verbindet, gebildet sind;
einen Behälter (BO), der innerhalb des Gehäuses (1100) angeordnet ist, um eine Flüssigkeit (LQ) zu enthalten, wobei ein Einlass des Behälters (BO) in Kontakt mit dem ersten Luftpfad (AP1) ist;
einen Gebläseventilator (1200), der in dem zweiten Luftpfad (AP2) angeordnet ist, wobei der Gebläseventilator (1200) erzwingt, dass Luft von dem zweiten Lufteinlass (AI2) zu dem Mischraum (MS) strömt;
einen Ozongenerator (1300), der in dem zweiten Luftpfad (AP2) angeordnet ist, um unter Verwendung von durch den zweiten Lufteinlass (AI2) eingeführter Luft Ozon zu erzeugen; und
**gekennzeichnet durch**
einen Mischventilator (1400), der in dem Mischraum (MS) angeordnet ist, wobei der Mischventilator (1400) Luft, die in dem Behälter (BO) verdampfte Flüssigkeit enthält und durch den ersten Luftpfad (AP1) strömt, um an dem Mischraum (MS) anzukommen, und Luft, die Ozon enthält und durch den zweiten Luftpfad (AP2) strömt, um an dem Mischraum (MS) anzukommen, mischt.

2. Luftsterilisator nach Anspruch 1, wobei der erste Luftpfad (AP1) und der zweite Luftpfad (AP2) nicht parallel zueinander sind.

3. Luftsterilisator nach Anspruch 1, wobei der erste Luftpfad (AP1) und der zweite Luftpfad (AP2) senkrecht zueinander angeordnet sind.

4. Luftsterilisator nach Anspruch 1, wobei der erste Luftpfad (AP1) einen Abschnitt mit einem relativ kleineren Durchmesser verglichen mit anderen Abschnitten aufweist, mit dem ein Einlass des Behälters (BO) in Kontakt ist, so dass Flüssigkeit in dem Behälter (BO) aufgesaugt werden kann.

5. Luftsterilisator nach Anspruch 1, ferner umfassend:
eine UV-LED (1600), die in dem dritten Luftpfad (AP3) angeordnet ist, und wobei wenigstens ein Abschnitt einer Innenwand des dritten Luftpfads (AP3) mit einem TiO₂- oder Delafossit-Überzug (1100a) gebildet ist.

6. Luftsterilisator nach Anspruch 1, ferner umfassend:
einen Gebläseventilatorantriebsmotor, der den Gebläseventilator dreht; und
wobei der Mischventilator (1400) mittels durch den zweiten Luftpfad (AP2) eingeführter Luft gedreht wird.

7. Luftsterilisator nach Anspruch 6, wobei ein Durchmesser des zweiten Luftpfads (AP2) sich allmählich verringert, um mit dem Mischraum (MS) verbunden zu sein, um eine Geschwindigkeit von in den Mischraum (MS) von dem zweiten Luftpfad (AP2) strömender Luft zu erhöhen, um die Drehgeschwindigkeit des Mischventilators (1400) zu erhöhen.

8. Luftsterilisator nach Anspruch 1, ferner umfassend:
einen Gebläseventilatorantriebsmotor, der den Gebläseventilator (1200) dreht; und
einen Mischventilatorantriebsmotor, der den Mischventilator (1400) dreht.

9. Luftsterilisator nach Anspruch 8, ferner umfassend:
eine Steuereinrichtung (1500), welche die Drehgeschwindigkeit des Gebläseventilatorantriebsmotors und die Drehgeschwindigkeit des Mischventilatorantriebsmotors individuell steuert, um ein Mischverhältnis von Luft, die in dem Behälter (BO) verdampfte Flüssigkeit enthält, die durch den ersten Luftpfad (AP1) hindurchgeht, um an dem Mischraum (MS) anzukommen, und von Luft, die Ozon enthält, die durch den zweiten Luftpfad (AP2) hindurchgeht, um an dem Mischraum (MS) anzukommen, zu steuern.

10. Luftsterilisator nach Anspruch 9, wobei der Behälter (BO) eine Limonenlösung (Olefin) enthält, und
die Steuereinrichtung (1500) die Geschwindigkeit des Gebläseventilatorantriebsmotors und des Mischventilatorantriebsmotors individuell steuert, so dass ein Verhältnis von Ozon : Limonen in einem Bereich von 1 : 0,3 ~ 1 : 3 ist.

11. Luftsterilisator nach Anspruch 9, wobei der Behälter (BO) Wasserstoffperoxyd enthält, und
die Steuereinrichtung (1500) die Geschwindigkeit des Gebläseventilatorantriebsmotors und des Mischventilatorantriebsmotors individuell steuert, so dass ein Verhältnis von Ozon : Wasserstoffperoxyd in einem Bereich von 1 : 0,3 ~ 1 : 2 ist.

12. Luftsterilisator nach Anspruch 8, wobei der Mischventilator (1400) mit dem Mischventilatorantriebsmotor trennbar verbunden ist.

13. Luftsterilisator nach Anspruch 1, ferner umfassend:
eine Vorrichtung zum Messen eines Flüssigkeitsniveaus (100) zum Messen einer verbleibenden Menge an Flüssigkeit in dem Behälter, wobei die Vorrichtung zum Messen eines Flüssigkeitsniveaus (100) umfasst:
eine Lichtemittierungsvorrichtung (111); und
eine Lichtempfangsvorrichtung (121), die an wenigstens einer einer ersten Position (P1), an der ein durch die Lichtemittierungsvorrichtung (121) erzeugtes Licht durch einen Behälter (BO) mit Flüssigkeit (LQ) hindurchgeht, um anzukommen, und einer zweiten Position (P2) angeordnet ist, an der ein durch die Lichtemittierungsvorrichtung (111) erzeugtes Licht durch einen leeren Behälter (BO) hindurchgeht, um anzukommen.

14. Luftsterilisator nach Anspruch 13, wobei eine Anzahl der Lichtemittierungsvorrichtung (111) und der Lichtempfangsvorrichtung (121) eine Mehrzahl ist, und
die Lichtemittierungsvorrichtungen (111) und die Lichtempfangsvorrichtungen (121) in der Z-Achsen-Richtung (Gravitätsrichtung) angeordnet sind.

15. Luftsterilisator nach Anspruch 13, wobei die Lichtemittierungsvorrichtung (311) ein Licht erzeugt, dessen Querschnitt eine vertikale lineare Form aufweist.

## Revendications

1. Un stérilisateur d'air (1000, 2000) comprenant :
un boîtier (1100) dans lequel sont formés un premier chemin d'air (AP1) reliant une première entrée d'air (AI1) et un espace de mélange (MS), un deuxième chemin d'air (AP2) reliant une deuxième entrée d'air (AI2) et l'espace de mélange (MS), et un troisième chemin d'air (AP3) reliant l'espace de mélange (MS) et une sortie d'air (AO) ;
un récipient (BO) disposé à l'intérieur du boîtier (1100) pour contenir un liquide (LQ), une entrée du récipient (BO) étant en contact avec le premier chemin d'air (AP1) ;
un ventilateur soufflant (1200) disposé dans le deuxième chemin d'air (AP2), le ventilateur soufflant (1200) forçant l'air à s'écouler de la deuxième entrée d'air (AI2) vers l'espace de mélange (MS) ;
un générateur d'ozone (1300) disposé dans le deuxième chemin d'air (AP2) pour générer de l'ozone en utilisant l'air introduit par la deuxième entrée d'air (AI2) ; et
**caractérisé par** un ventilateur mélangeur (1400) disposé dans l'espace de mélange (MS), le ventilateur mélangeur (1400) mélangeant de l'air contenant du liquide évaporé dans le récipient (BO) et circulant à travers le premier chemin d'air (AP1) pour arriver à l'espace de mélange (MS) et de l'air contenant de l'ozone et circulant à travers le deuxième chemin d'air (AP2) pour arriver à l'espace de mélange (MS).

2. Le stérilisateur d'air selon la revendication 1, dans lequel le premier chemin d'air (AP1) et le deuxième chemin d'air (AP2) ne sont pas parallèles l'un à l'autre.

3. Le stérilisateur d'air selon la revendication 1, dans lequel le premier chemin d'air (AP1) et le deuxième chemin d'air (AP2) sont disposés perpendiculairement l'un à l'autre.

4. Le stérilisateur d'air selon la revendication 1, dans lequel le premier chemin d'air (AP1) a une partie de diamètre relativement plus petit par rapport aux autres parties, avec laquelle une entrée du récipient (BO) est en contact, de sorte que le liquide dans le récipient (BO) est apte à être aspiré.

5. Le stérilisateur d'air selon la revendication 1, comprenant en outre :
une DEL UV (1600) disposée dans le troisième chemin d'air (AP3), et au moins une partie de la paroi interne du troisième chemin d'air (AP3) étant formée avec un revêtement de TiO₂ ou de Delafosite (1100a).

6. Le stérilisateur d'air selon la revendication 1, comprenant en outre :
un moteur d'entraînement de ventilateur soufflant faisant tourner le ventilateur soufflant ; et
le ventilateur mélangeur (1400), mis en rotation par l'air introduit à travers le deuxième chemin d'air (AP2).

7. Le stérilisateur d'air selon la revendication 6, dans lequel le diamètre du deuxième chemin d'air (AP2) est progressivement réduit pour être connecté à l'espace de mélange (MS), afin d'augmenter la vitesse de l'air s'écoulant dans l'espace de mélange (MS) depuis le deuxième chemin d'air (AP2) afin d'augmenter la vitesse de rotation du ventilateur mélangeur (1400).

8. Le stérilisateur d'air selon la revendication 1, comprenant en outre :
un moteur d'entraînement du ventilateur soufflant faisant tourner le ventilateur soufflant (1200) ; et
un moteur d'entraînement du ventilateur de mélange faisant tourner le ventilateur de mélange (1400).

9. Le stérilisateur d'air selon la revendication 8, comprenant en outre :
un contrôleur (1500) commandant individuellement la vitesse de rotation du moteur d'entraînement du ventilateur de soufflage et la vitesse de rotation du moteur d'entraînement du ventilateur de mélange pour commander le rapport de mélange de l'air contenant le liquide évaporé dans le récipient (BO), qui passe à travers le premier chemin d'air (AP1) pour arriver à l'espace de mélange (MS), et de l'air contenant de l'ozone, qui traverse le deuxième chemin d'air (AP2) pour arriver à l'espace de mélange (MS).

10. Le stérilisateur d'air selon la revendication 9, dans lequel le récipient (BO) contient une solution de limonène (oléfine), et
le contrôleur (1500) commande la vitesse du moteur d'entraînement du ventilateur de soufflage et du moteur d'entraînement du ventilateur de mélange individuellement de telle sorte qu'un rapport ozone : limonène est compris dans une gamme allant de 1 : 0,3 à 1 : 3.

11. Le stérilisateur d'air selon la revendication 9, dans lequel le récipient (BO) contient du peroxyde d'hydrogène, et
le contrôleur (1500) commande individuellement la vitesse du moteur d'entraînement du ventilateur de soufflage et du moteur d'entraînement du ventilateur de mélange de telle sorte qu'un rapport ozone : peroxyde d'hydrogène soit dans une gamme allant de 1 : 0,3 à 1 : 2.

12. Le stérilisateur d'air selon la revendication 8, dans lequel le ventilateur de mélange (1400) est connecté de manière déconnectable au moteur d'entraînement du ventilateur de mélange.

13. Le stérilisateur d'air selon la revendication 1, comprenant en outre :
un appareil pour mesurer le niveau de liquide (100) pour mesurer une quantité restante de liquide dans le récipient, l'appareil pour mesurer le niveau de liquide (100) comprenant :
un dispositif (111) émetteur de lumière ; et
un dispositif (121) récepteur de lumière disposé au niveau d'au moins une parmi une première position (P1), en laquelle une lumière générée par le dispositif (111) émetteur de lumière traverse un récipient (BO) avec un liquide (LQ) jusqu'à arriver, et une deuxième position (P2), en laquelle une lumière générée par le dispositif (111) émetteur de lumière traverse un récipient vide (BO) jusqu'à arriver.

14. Le stérilisateur d'air selon la revendication 13, dans lequel les dispositifs (111) émetteurs de lumière et les dispositifs (121) récepteurs de lumière sont en pluralité, et les dispositifs (111) émetteurs de lumière et les dispositifs (121) récepteurs de lumière sont agencés dans la direction d'axe Z (direction de la gravité).

15. Le stérilisateur d'air selon la revendication 13, dans lequel le dispositif (311) émetteur de lumière génère une lumière dont la section transversale a une forme linéaire verticale.
